# NEUE EUROPÄISCHE PATENTSCHRIFT

(11) **EP 2 883 472 B2**
(45) Veröffentlichungstag und Bekanntmachung des Hinweises auf die Entscheidung über den Einspruch: **26.12.2018**
(45) Hinweis auf die Patenterteilung: 13.04.2016
(21) Anmeldenummer: 13197231.7
(22) Anmeldetag: 13.12.2013
(51) Int. Cl.: A61F 13/62, A44B 18/00, D04B 21/04

(54) **Verbundstoffelement für Klettverschlüsse sowie Verfahren zur Bildung eines Verbundstoffelementes von Klettverschlüssen**
Compound material element for Velcro elements and method for forming a compound material element from Velcro elements
Élément composite pour fermetures velcro et procédé de formation d'un élément composite de fermetures velcro

(43) Veröffentlichungstag der Anmeldung: 17.06.2015
(73) Patentinhaber: Mondi Gronau GmbH, 48599 Gronau (DE)
(72) Erfinder: Homölle, Dieter, 48607 Ochtrup (DE); Baldauf, Georg, 48366 Laar (DE)
(74) Vertreter: von dem Borne, Andreas

(56) Entgegenhaltungen:
- EP-A1- 0 694 642
- EP-A1- 1 690 967
- EP-A1- 2 439 323
- EP-A1- 2 455 522
- EP-A2- 0 548 050
- EP-A2- 2 191 801
- EP-A2- 2 191 801
- EP-B1- 0 548 050
- EP-B1- 1 579 779
- EP-B1- 1 690 967
- WO-A1-00/42964
- WO-A1-00/42964
- WO-A1-97/38656
- WO-A2-2004/050970
- DE-A1- 19 506 037
- DE-A1- 19 506 038
- DE-A1- 29 609 782
- DE-T2- 69 503 437
- JP-A- 2003 325 208
- US-A- 5 019 073
- US-A1- 2004 022 993
- US-A1- 2004 022 993
- US-A1- 2004 099 019
- US-A1- 2004 099 019

## Beschreibung

Die Erfindung betrifft ein Verbundstoffelement für Klettverschlüsse, insbesondere für einen Windelverschluss, mit einem Träger und einem auf den Träger kaschierten Textilmaterial, welches eine aus Fäden gewirkte Grundstruktur und in die Grundstruktur durch Wirken eingebundene Schlaufenfäden aufweist, welche zur Verbindung mit Kletthaken vorgesehene Schlaufen bilden. Die Erfindung betrifft des Weiteren ein Verfahren zur Bildung eines Verbundstoffelementes von Klettverschlüssen.

Klettverschlüsse bestehen gemäß ihrer üblichen Ausgestaltung aus einem Schlaufenteil sowie einem Hakenteil. Entsprechende Klettverschlüsse werden auch als Hook-and-Loop-Systeme bezeichnet. Klettverschlüsse werden sowohl für die mehrfache Verwendung als auch für Wegwerfartikel wie Windeln, insbesondere Babywindeln oder Inkontinenzartikel für Erwachsene, verwendet.

Bei Windelverschlüsse für Einwegwindeln werden in der Praxis meist Verbundstoffelemente aus einer bedruckten Polyethylenfolie als Träger und einem darauf mit Klebstoff kaschierten Gewirke aus Polyamid als Schlaufenfolie eingesetzt. Das Verbundstoffelement kann beispielsweise an einem Taillenbereich auf Höhe des Bauches oder Unterleibs eines Benutzers angeordnet sein, um zwei die Taille seitlich umgreifende Hakenteile aufzunehmen. Das Verbundstoffelement wird in diesem Zusammenhang in der Praxis teils auch als "landing zone" bezeichnet.

Gerade bei Wegwerfprodukten wie Windeln werden insgesamt geringe Produktionskosten und damit auch geringe Flächengewichte von Träger und dem darauf kaschierten Textilmaterial angestrebt, so dass das Textilmaterial bei einer Ausgestaltung als Gewirke eine offene und luftige Struktur aufweist.

Um sowohl geringe Produktionskosten als auch eine gute Klettwirkung zu erreichen, können der Träger und das Textilmaterial in einem Muster aus Klebeflächen und klebstofffreien Bereichen miteinander verbunden werden. Entsprechend ausgeführte Verbundstoffelemente sind aus der EP 1 579 779 B1 sowie der EP 1 690 967 B1 bekannt, wobei gemäß der EP 1 690 967 B1 um den Rand der einzelnen Verbundstoffelemente der Klebstoff rahmenförmig angeordnet ist, um dort ein Ausreißen zu vermeiden.

Die aus dem Stand der Technik bekannten Verbundstoffelemente lassen sich mit einem geringen Flächengewicht und damit kostengünstig herstellen. Es ergibt sich aber der Nachteil, dass die offene Struktur des Gewirkes mit der darunter liegenden Trägerfolie eine unerwünschte, wenig weiche Optik und Haptik aufweist.

Des Weiteren sind aus dem Stand der Technik Verbundstoffelemente mit einer Oberfläche aus Nonwoven bekannt, wobei das Nonwoven Schlaufen für die Verankerung von Kletthaken bildet. Um allerdings eine ausreichende Klettwirkung sowie Festigkeit zu erreichen, ist ein vergleichsweise großes Flächengewicht von typischerweise 40 bis 50 g/m² notwendig. Eine angenehme Oberfläche mit einem Softtouch-Effekt ist dabei also aufgrund des größeren Flächengewichtes mit relativ hohen Herstellungskosten verbunden. Ein Verbundstoffelement mit einem für die Verbindung mit Kletthaken vorgesehenen Nonwoven ist aus der WO 03/105621 A1 bekannt.

Aus der EP 2 191 801 A2, der WO 00/42964 A1 sowie der US 2004/022993 A1 sind ebenfalls Verbundstoffelemente mit einer Oberfläche aus Nonwoven bekannt, wobei in das Nonwoven zusätzliche Schlaufen eingewirkt sind. Da als textile Grundstruktur ein Nonwovenmaterial eingesetzt wird, weist das gesamte Verbundstoffelement eine weiche angenehme Haptik auf wobei aber aufgrund des Einsatzes von Nonwoven ein vergleichsweise großes Flächengewicht und damit große Herstellungskosten resultieren.

Aus der US 2004/0099019 A1 ist ein Verbundstoffelement bekannt, welches einen bedruckten Träger sowie ein auf den Träger kaschiertes textiles Material aufweist. Das textile Material weist eine aus Fäden gewirkte Grundstruktur und in die Grundstruktur durch Wirken eingebundene Schlaufenfäden auf, welche zur Verbindung mit Kletthaken vorgesehene Schlaufen bilden. Die Schlaufenfäden können aus Bi-Komponenten Fasern oder einem entlang seiner Längsrichtung trennbaren Garn gebildet sein. Diese gattungsgemäße Entgegenhaltung beschäftigt sich mit einer Verbesserung der Sichtbarkeit eines Aufdruckes, weshalb das textile Material nicht zu dicht sein darf.

Der vorliegenden Erfindung liegt die Aufgabe zugrunde, ein Verbundstoffelement anzugeben, welches durch Wirken gebildete Schlaufen aufweist und bei einem geringen Flächengewicht mit einer weichen Oberfläche versehen ist. Des Weiteren soll ein Verfahren zur Bildung eines solchen Verbundstoffelementes angegeben werden.

Gegenstand der Erfindung und Lösung der Aufgabe sind ein Verbundstoffelemente gemäß Patentanspruch 1 sowie ein Verfahren gemäß Patentanspruch 11.

Um eine möglichst einfache Herstellung bei einem geringen Flächengewicht zu erreichen, ist im Rahmen der Erfindung vorgesehen, dass die Grundstruktur gewirkt ist. Die Grundstruktur ist aus Fäden eines ersten Grundgarns und aus Fäden eines zweiten Grundgarns gewirkt. Davon ausgehend ist bevorzugt vorgesehen, dass das erste Grundgarn und das zweite Grundgarn aus Polyamid, beispielsweise PA6 oder PA66, gebildet sind. Ausgehend von einem eine gewirkte Grundstruktur aufweisenden Verbundstoffelement mit den eingangs beschriebenen Merkmalen ist erfindungsgemäß vorgesehen, dass die Schlaufenfäden von einem texturierten Multifilamentgarn insbesondere Multifilamentgarn auf der Basis von Polyester oder Polyamid gebildet sind.

Durch den Einsatz eines texturierten Multifilamentgarns für die Schlaufenfäden wird eine besonders weiche, weniger offene Oberfläche erreicht, welche sich durch die Optik einer gleichmäßigen, textilähnlichen Schicht und auch eine weichere Haptik auszeichnet. Insbesondere kann eine Optik mit einer weichen Anmutung erreicht werden, welche in etwa des Aussehens einer Außenschicht üblicher Windeln entspricht, welche als "backsheet" bezeichnet wird.

Erfindungsgemäß weist das Multifilamentgarn gegenüber bekannten Verbundstoffelementen mit eingewirkten Schlaufen eine erhöhte Anzahl an Filamenten auf. So ist erfindungsgemäß vorgesehen, dass das texturierte Multifilamentgarn der Schlaufenfäden bei einer Feinheit zwischen 35 dtex und 55 dtex zwischen 11 bis 25, insbesondere zwischen 13 und 20, texturierte Filamente aufweist.

Im Gegensatz zu aus dem Stand der Technik bekannten Verbundstoffelementen mit einem vollständig durch Wirken gebildeten Textilmaterial tritt auch bei einem geringen Flächengewicht weniger stark eine Gitterstruktur hervor, die durch die in Produktionsrichtung verlaufenden Kettstränge und die quer dazu verlaufenden Maschenreihen gebildet ist.

Die texturierten Multifilamentgarne füllen die freien Zwischenräume des lockeren, offenen Textilmaterials besser aus. Für den Benutzer erscheint das Verbundstoffelement damit hochwertiger.

Auch wenn das Verbundstoffelement für einen Klettverschluss in der Regel so angeordnet wird, dass bei einer normalen Benutzung kein Kontakt zu der Haut eines Benutzers vorliegt, kann ein solcher Kontakt in der Praxis nicht immer verhindert werden. Durch die weichere Haptik ergibt sich bei der erfindungsgemäßen Ausgestaltung damit auch ein geringeres Risiko einer Hautreizung oder ähnlichen Beeinträchtigungen eines Benutzers.

Durch die Texturierung wird dem zunächst glatten Multifilamentgarn bei der Garnherstellung je nach Grad der Texturierung eine wellige, gebogene oder auch gekräuselte Struktur verliehen. Dem Multifilamentgarn auf der Basis von Polyester oder Polyamid wird damit eine Struktur verliehen, welche eher für Naturfasern typisch ist.

Für die Kräuselung bzw. Texturierung kommen grundsätzlich unterschiedliche Verfahren in Betracht. Die Verformung der einzelnen Filamente des Multifilamentgarns kann rein mechanisch durch ein Blasverfahren und/oder mittels Walzen oder auch kombiniert mechanisch und thermisch erfolgen. Im Hinblick auf die vorliegende Erfindung ist eine Verwirbelung und Verdrillung mittels eines Fluidstroms, beispielsweise mit Luftdüsen, besonders geeignet, um eine feine, weiche Texturierung des Multifilamentgarns für die Schlaufenfäden zu erreichen.

Für das textunierte Multifilamentgarn der Schlaufenfäden kommen unterschiedliche Polymere wie beispielsweise Polyester, Polyamid, Polyactide und Polypropylen sowie auch Polymermischungen in Betracht.

Besonders bevorzugt ist das texturierte Multifilamentgarn auf der Basis von Polyethylenterephthalat (PET) gebildet, welches sich durch gute mechanische Eigenschaften auszeichnet und auch gut texturiert werden kann.

Erfindungsgemäß ist vorgesehen, dass für die Herstellung des Textilmaterials texturiertes Multifilamentgarn für die Schlaufenfäden eingesetzt wird. Optional kann auch vorgesehen sein, dass die Schlaufen auch nach der Kaschierung des Textilmaterials mit dem Träger mechanisch bearbeitet sind, um die Schlaufen aufzurichten, die einzelnen Filamente weiter voneinander zu trennen oder auch an den einzelnen Filamenten eine noch stärkere Verdrillung oder Kräuselung hervorzurufen. Zu diesem Zweck kann auch das gebildete Verbundstoffmaterial beispielsweise mit einem Luftstrom oder mit Bürsten bearbeitet werden.

Nicht erfindungsgemäss, aber möglich ist es, dass die Grundstruktur aus einem Nonwoven-Material gebildet ist, wobei dann die Schlaufenfäden in die Grundstruktur aus Nonwoven eingewirkt sind. Im Rahmen einer solchen Ausgestaltung ergibt sich eine besonders gleichmäßige, weiche Struktur, wobei letztlich die bei einem vollständig gewirkten textilen Material typische gitterartige Struktur von Kettsträngen (Maschenstäbchen) und Maschenreihen nicht oder nur kaum erkennbar ist.

Die Grundstruktur ist zunächst dazu vorgesehen, um die Schlaufenfäden aufzunehmen und zu halten. Die Grundstruktur kann dazu beispielsweise aus Monofilamentfäden gebildet werden, die typischerweise eine Feinheit zwischen 15 und 30 dtex, beispielsweise 22 dtex, aufweisen.

Darüber hinaus kann aber auch die Grundstruktur zumindest in einem gewissen Maße die optischen und haptischen Eigenschaften beeinflussen.

Gemäß einer Variante der Erfindung ist deshalb vorgesehen, dass das erste Grundgarn von einem Monofilamentgarn mit einer Feinheit zwischen 15 und 30 dtex und das zweite Grundgarn von einem bevorzugt nichttexturierten Multifilamentgarn mit einer Feinheitzwischen 35 und 55 dtex gebildet werden.

Die Feinheit des texturierten Multifilamentgarns für die Schlaufenfäden sowie des ersten Grundgarns und des zweiten Grundgarns ist anhand der mechanischen, optischen und haptischen Anforderungen an das Verbundstoffelement auszuwählen. Darüber hinaus bestimmen die Eigenschaften der Garne und die Dichte des gebildeten Textilmaterials wesentlich die Kletteigenschaften. Im Hinblick auf den Einsatz als Teil eines Klettverschlusses von einem Wegwerfartikel sind dabei einander gegenläufige Aspekte zu beachten. Einerseits wird aus Kostengründen ein möglichst geringes Flächengewicht des Textilmaterials angestrebt. Andererseits muss das textile Material auch eine ausreichende Festigkeit und ausreichende Verbindungsmöglichkeiten für Kletthaken aufweisen.

Vor diesem Hintergrund weist das Textilmaterial vorzugsweise ein Flächengewicht zwischen 15 und 45 g/m², vorzugsweise zwischen 18 und 25 g/m² auf.

Auch dem beschriebenen Textilmaterial alleine kommt ein erfinderischer Überschuss zu. Die Erfindung betrifft damit auch das Textilmaterial wie beschrieben ohne dem Träger. Also ein Textilmaterial, welches eine aus Fäden oder Fasern gebildete Grundstruktur und in die Grundstruktur durch Wirken eingebundene Schlaufenfäden aufweist, welche zur Verbindung mit Kletthaken vorgesehene Schlaufen bilden, wobei die Schlaufenfäden von einem texturierten Multifilamentgarn gebildet sind.

Als Träger kommt im Rahmen der Erfindung neben einer üblichen Folie grundsätzlich auch ein Nonwoven-Material in Betracht, wobei der Träger häufig mit einem Dekor bedruckt ist, welches dann durch das aufkaschierte Textilmaterial sichtbar ist. Wenn der Träger gemäß einer bevorzugten Ausgestaltung der Erfindung aus einer ein- oder mehrschichtigen Folie gebildet ist, weist diese vorzugsweise zumindest an der Fläche, die mit dem Textilmaterial verklebt ist, ein Polyolefin, insbesondere Polyethylen als Hauptbestandteil auf. Aus Kostengründen ist insbesondere eine Polyethylen-Monofolie geeignet, wobei die Folie - unabhängig von ihrer konkreten Ausgestaltung - vorzugsweise eine Dicke zwischen 8 µm und 30 µm aufweist.

Bei dem Einsatz einer Folie als Träger kann ein Dekoraufdruck in einfacher Weise auf die Folie gedruckt werden. Vorzugsweise ist der Dekoraufdruck gegenüberliegend des Textilmaterials angeordnet und dann durch die Folie und das bei der Anordnung an einer Windel außen liegende Textilmaterial sichtbar.

Der Träger und das Textilmaterial sind vorzugsweise durch Klebstoff miteinander verbunden. Dabei kann der Klebstoff nicht vollflächig in einem Muster angeordnet sein, wobei das Muster Klebeflächen und klebstofffreie Bereiche aufweist. Zusätzlich kann entsprechend der Form des Verbundstoffelementes auch ein umlaufender Klebstoffrahmen vorgesehen sein, wobei entsprechende Ausgestaltungen in der EP 1 690 967 B1 beschrieben sind. Vorzugsweise beträgt die Flächenabdeckung etwa 20 %. Beispielsweise kann der Klebstoff in einem Muster aus sich kreuzenden, wellenförmig überlaufenden Klebstoffstreifen gebildet sein, wobei ein solches Muster auch als "dog bone"-Muster bezeichnet wird. Ein entsprechendes Muster ist beispielsweise aus der Fig. 2a der EP 2 439 323 A1 bekannt.

Das Auftragsgewicht des Klebstoffes beträgt (bezogen auf die Gesamtfläche von Klebeflächen und klebstofffreien Bereichen) vorzugsweise etwa 2 g/m².

Gegenstand der Erfindung ist auch ein Verfahren zur Bildung eines Verbundstoffelementes von Klettverschlüssen, wobei eine Folie als Träger zugeführt wird, wobei ein Textilmaterial zugeführt wird, welches eine aus Fäden gewirkte Grundstruktur und in die Grundstruktur durch Wirken eingebundene Schlaufenfäden aufweist, welche zur Verbindung mit Kletthaken vorgesehene Schlaufen bilden und von einem texturierten Multifilamentgarn, insbesondere einem Multifilamentgarn, auf der Basis von Polyester oder Polyamid, gebildet sind, wobei der Träger und das Textilmaterial durch ein in einem Muster aus Klebeflächen und klebstofffreien Bereichen angeordneten Klebstoff zu einer Materialbahn verbunden werden und wobei aus der Materialbahn einzelne Verbundstoffelemente abgetrennt werden.

Zusätzlich zu dem Einsatz von texturiertem Multifilamentgarn für die Schlaufenfäden kann optional auch nach der Bildung der Materialbahn durch Druckluft, Bürsten oder eine andere mechanische Behandlung das Textilmaterial zusätzlich aufgebauscht werden.

Des Weiteren ist vorgesehen, dass von der Materialbahn Verbundstoffelemente abgetrennt und jeweils auf einem Taillenbereich einer Windel angeordnet werden.

Des Weiteren erfolgt auch ein Bedrucken des Trägers in Form einer Folie.

Die Erfindung wird im Folgenden anhand eines Ausführungsbeispiels unter Bezugnahme auf die Figuren erläutert. Es zeigen:
- Fig. 1: ein Verbundstoffelement gemäß dem Stand der Technik,
- Fig. 2: ein erfindungsgemäßes Verbundstoffelement.

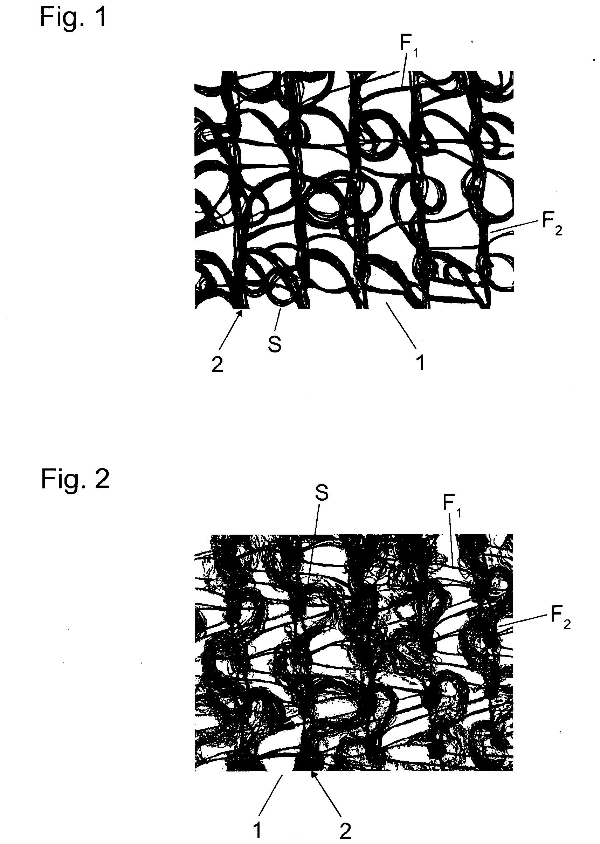

Die Fig. 1 zeigt ein bekanntes Verbundstoffelement für Klettverschlüsse, insbesondere für einen Windelverschluss, mit einem Träger 1 in Form einer Folie und einem auf den Träger 1 kaschierten Textilmaterial 2. Das Textilmaterial 2 ist ein Gewirke, welches eine gewirkte Grundstruktur aus Fäden F₁ eines ersten Grundgarns und Fäden F₂ eines zweiten Grundgarns aufweist.

In die Grundstruktur sind durch Wirken des Weiteren Schlaufenfäden S eingebunden, welche zur Verbindung mit Kletthaken vorgesehene Schlaufen bilden.

Der Träger 1 und das Textilmaterial 2 sind durch einen nicht dargestellten Klebstoff verbunden, der in einem Muster mit Klebeflächen und klebstofffreien Bereichen angeordnet ist. Geeignet ist beispielsweise eine aus der Fig. 2a der EP 2 439 323 A1 bekannte "dog bone"-Struktur mit einander kreuzenden, wellenförmigen Klebstoffsträngen, wobei die Flächenabdeckung der Klebeflächen vorzugsweise bei etwa 20 % und das über die gesamte Fläche gemittelte Auftragsgewicht bei 2 g/m² liegt. Geeignet sind beispielsweise 1-Komponenten-Klebstoffe auf der Basis von Polyurethan, welche insbesondere durch Luftfeuchtigkeit und/oder Wasserberieselung aushärten.

Bei dem Ausführungsbeispiel der Fig. 1 gemäß dem Stand der Technik sind die Fäden F1, F2 des ersten Grundgarns und des zweiten Grundgarns aus nichttexturierten PA6-Monofilamenten mit einer Feinheit von 22 dtex gebildet, wobei für die Schlaufenfäden ein nichttexturiertes PA6-Multifilamentgarn mit neun Filamenten vorgesehen ist.

Die Fig. 2 zeigt dagegen ein erfindungsgemäßes Verbundstoffelement für Klettverschlüsse, bei dem die Schlaufenfäden S von einem texturierten Multifilamentgarn gebildet sind. Es ist zu erkennen, dass die einzelnen Filamente der Schlaufenfäden S stark gekräuselt und aufgebauscht sind, so dass sich eine besonders gleichmäßig weiche Optik und Haptik ergibt. Die in der Fig. 1 deutlich sichtbare gitterartige Struktur mit Kettsträngen (Maschenstäbchen) und quer dazu verlaufenden Maschenreihen ist weniger stark erkennbar.

Im Rahmen des Ausführungsbeispiels sind die Schlaufenfäden aus Polyethylenterephthalat (PET) mit einer Feinheit von 44 dtex gebildet, wobei das texturierte Multifilamentgarn 13 Filamente aufweist.

Während das erste Grundgarn wie zuvor von einem Faden F₁ in Form eines Monofilamentgarns mit einer Feinheit von 22 dtex gebildet ist, handelt es sich bei dem Faden F₂ des zweiten Grundgarns um ein nichttexturiertes Monofilamentgarn mit einer Feinheit von 44 dtex. Die Fäden F₁ und F₂ des ersten Grundgarns sowie des zweiten Grundgarns sind aus PA6 gebildet, so dass in dem beschriebenen Ausführungsbeispiel das textile Material des erfindungsgemäßen Verbundstoffelementes nicht sortenrein ist. Grundsätzlich kommt es im Rahmen der Erfindung aber auch in Betracht, die Schlaufenfäden S aus einem texturierten Polyamid-Multifilamentgarn oder aus texturiertem Multifilamentgarn auf Basis eines anderen Polymers zu bilden.

Ausgehend von einem Verbundstoffelement gemäß der Fig. 2 wurde zur Überprüfung der funktionellen Eigenschaften durch eine Veränderung der Maschenweite des Textilmaterials 2 sowie einer geeigneten Variation der Feinheit das Flächengewicht in einem Bereich von 18 bis 35 g/m² variiert. In dem gesamten Bereich wurde ein funktioneller Windelverschluss erreicht, der ausreichend hohe Peel- und Scherwerte aufweist.

## Patentansprüche

1. Verbundstoffelement für Klettverschlüsse, insbesondere für einen Windelverschluss, mit einem Träger (1) und einem auf den Träger kaschierten Textilmaterial (2), welches eine aus Fäden (F₁, F₂) gewirkte Grundstruktur und in die Grundstruktur durch Wirken eingebundene Schlaufenfäden (S) aufweist, welche zur Verbindung mit Kletthaken vorgesehene Schlaufen bilden, wobei die Schlaufenfäden (S) von einem texturierten Multifilamentgarn gebildet sind,
**dadurch gekennzeichnet, dass** das texturierte Multifilamentgarn der Schlaufenfäden (S) eine Feinheit zwischen 35 dtex und 55 dtex aufweist und aus 11 bis 25 Filamenten gebildet ist
**und dass die Grundstruktur aus Fäden (F₁) eines ersten Grundgarns und Fäden (F₂) eines zweiten Grundgarns gewirkt ist.**

2. Verbundstoffelement nach Anspruch 1, **dadurch gekennzeichnet, dass** das texturierte Multifilamentgarn der Schlaufenfäden (S) auf Basis eines Polymers ausgewählt aus der Gruppe Polyethylenterephthalat (PET), Polyamid (PA), Polyactide (PLA) und Polypropylen (PP) gebildet ist.

3. Verbundstoffelement nach Anspruch 1 oder 2, **dadurch gekennzeichnet, dass** die Filamente des texturierten Multifilamentgarns der Schlaufenfäden (S) durch einen Luftstrom verdrillt sind.

4. Verbundstoffelement nach **einem der Ansprüche 1 bis 3, dadurch gekennzeichnet, dass** das erste Grundgarn und das zweite Grundgarn aus Polyamid gebildet sind.

5. Verbundstoffelement nach **einem der Ansprüche 1 bis 4**, **dadurch gekennzeichnet, dass** das erste Grundgarn von einem Monofilamentgarn mit einer Feinheit zwischen 15 und 30 dtex und das zweite Grundgarn von einem Multifilamentgarn mit einer Feinheit zwischen 35 und 55 dtex gebildet sind.

6. Verbundstoffelement nach einem der Ansprüche 1 bis **5**, **dadurch gekennzeichnet, dass** das Textilmaterial (2) ein Flächengewicht zwischen 15 und 45 Gramm pro Quadratmeter (g/m²) aufweist.

7. Verbundstoffelement nach einem der Ansprüche 1 bis **6, dadurch gekennzeichnet, dass** der Träger (1) eine Folie ist.

8. Verbundstoffelement nach Anspruch **7**, **dadurch gekennzeichnet, dass** die Folie einen Dekoraufdruck aufweist.

9. Verbundstoffelement nach Anspruch **7** oder **8, dadurch gekennzeichnet, dass** die Folie eine Dicke zwischen 8 µm und 30 µm aufweist.

10. Verbundstoffelement nach einem der Ansprüche 1 bis **9**, **dadurch gekennzeichnet, dass** der Träger (1) und das Textilmaterial (2) durch Klebstoff nicht vollflächig verbunden sind, wobei der Klebstoff in einem Muster aus Klebeflächen und klebstofffreien Bereichen angeordnet ist.

11. Verfahren zur Bildung eines Verbundstoffelementes von Klettverschlüssen,
wobei eine Folie als Träger (1) zugeführt wird,
wobei ein Textilmaterial (2) zugeführt wird, welches eine aus Fäden (F₁, F₂) gewirkte Grundstruktur und in die Grundstruktur durch Wirken eingebundene Schlaufenfäden (S) aufweist, welche zur Verbindung mit Kletthaken vorgesehene Schlaufen bilden und von einem 11 bis 25 Filamente sowie eine Feinheit zwischen 35 dtex und 55 dtex aufweisenden, texturierten Multifilamentgarn gebildet sind,
wobei **die Grundstruktur aus Fäden (F₁) eines ersten Grundgarns und Fäden (F₂) eines zweiten Grundgarns gewirkt ist.**
wobei der Träger (1) und das Textilmaterial (2) durch ein in einem Muster aus Klebeflächen und klebstofffreien Bereichen angeordneten Klebstoff zu einer Materialbahn verbunden werden und
wobei aus der Materialbahn einzelne Verbundstoffelemente abgetrennt werden.

12. Verfahren nach Anspruch **11**, wobei das texturierte Multifilamentgarn der Schlaufenfäden (S) nach der Bildung der Materialbahn zusätzlich durch Druckluft, Bürsten oder eine andere mechanische Behandlung des Textilmaterials zusätzlich aufgebauscht werden.

13. Verfahren nach Anspruch **11** oder **12**, wobei die von der Materialbahn abgetrennten Verbundstoffelemente auf einem Taillenbereich einer Windel angeordnet werden.

## Claims

1. Composite material element for hook-and-loop fasteners, in particular for a nappy closure, comprising a support (1) and a textile material (2) laminated onto the support, which textile material comprises a base structure knitted from threads (F₁, F₂) and loop threads (S) incorporated into the base structure by knitting, which form loops provided for connection to Velcro hooks, wherein the loop threads (S) are formed from a textured multifilament yarn, **characterized in that** the textured multifilament yarn of the loop threads (S) has a fineness between 35 dtex and 55 dtex and is formed from 11 to 25 filaments
and that the base structure is knitted from threads (F₁) of a first base yarn and threads (F₂) of a second base yarn.

2. The composite material element according to claim 1, **characterized in that** the textured mulifilament yarn of the loop threads (S) is formed on the basis of a polymer selected from the group of polyethylene terephthalate (PET), polyamide (PA), polyactide (PLA) and polypropylene (PP).

3. The composite material element according to claim 1 or 2, **characterized in that** the filaments of the textured multifilament yarn of the loop threads (S) are twisted by an air stream.

4. The composite material element according to any one of claims 1 to 3, **characterized in that** the first base yarn and the second base yarn are formed from polyamide.

5. The composite material element according to any one of claims 1 to 4, **characterized in that** the first base yarn is formed from a monofilament yarn having a fineness between 15 and 30 dtex and the second base yarn is formed from a multifilament yarn having a fineness between 35 and 55 dtex.

6. The composite material element according to any one of claims 1 to 5, **characterized in that** the textile material (2) has a grammage between 15 and 45 grams per square metre (g/m²).

7. The composite material element according to any one of claims 1 to 6, **characterized in that** the support (1) is a film.

8. The composite material element according to claim 7, **characterized in that** the film has a decorative imprint.

9. The composite material element according to claim 7 or 8, **characterized in that** the film has a thickness between 8 µm and 30 µm.

10. The composite material element according to any one of claims 1 to 9, **characterized in that** the support (1) and the textile material (2) are not joined by adhesive over the entire surface, wherein the adhesive is arranged in a pattern of adhesive areas and adhesive-free areas.

11. Method for forming a composite material element of hook-and-loop fasteners,
wherein a film is supplied as support (1),
wherein a textile material (2) is supplied which textile material comprises a base structure knitted from threads (F₁, F₂) and loop threads (S) incorporated into the base structure by knitting, which form loops provided for connection to Velcro hooks and which are formed from 11 to 25 filaments and textured multifilament yarn having a fineness between 35 dtex and 55 dtex,
wherein the base structure is knitted from threads (F₁) of a first base yarn and threads (F₂) of a second base yarn,
wherein the support (1) and the textile material (2) are joined to a material web by an adhesive arranged in a pattern of adhesive areas and adhesive-free areas and
wherein individual composite material elements are separated from the material web.

12. The method according to claim 11, wherein the textured multifilament yarn of the loop threads (S) after formation of the material web is additionally puffed up additionally by compressed air, brushing or another mechanical treatment of the textile material.

13. The method according to claim 11 or 12, wherein the composite material elements separated from the material web are arranged on a waist region of a nappy.

## Revendications

1. Élément composite pour fermetures auto-agrippantes, en particulier pour une fermeture de couche-culotte, comprenant un support (1) et un matériau textile (2) dissimulé sur le support, qui présente une structure de base produite par des brins (F₁, F₂) et des brins bouclés (S) intégrés par activation dans la structure de base, qui forment des boucles prévues pour être reliées à des crochets de crampon, sachant que les brins bouclés (S) sont formés par un fil multi-filaments texturé,
**caractérisé en ce que** le fil multi-filaments texturé des brins bouclés (S) présente une finesse entre 35 dtex et 55 dtex et est composé de 11 à 25 filaments
et **en ce que** la structure de base est produite par des brins (F₁) d'un premier fil de base et des brins (F₂) d'un second fil de base.

2. Élément composite selon la revendication 1,
**caractérisé en ce que** le fil multi-filaments texturé des brins bouclés (S) est fabriqué à base d'un polymère sélectionné parmi le groupe polyéthylène téréphtalate (PET), polyamide (PA), polyactide (PLA) et polypropylène (PP).

3. Élément composite selon la revendication 1 ou 2,
**caractérisé en ce que** les filaments du fil multi-filaments texturé des brins bouclés (S) sont torsadés par un flux d'air.

4. Élément composite selon l'une des revendications 1 à 3, **caractérisé en ce que** le premier fil de base et le second fil de base sont en polyamide.

5. Élément composite selon l'une des revendications 1 à 4, **caractérisé en ce que** le premier fil de base est formé d'un fil mono-filament ayant une finesse entre 15 et 30 dtex et le second fil de base est formé d'un fil multi-filaments ayant une finesse entre 35 et 55 dtex.

6. Élément composite selon l'une des revendications 1 à 5, **caractérisé en ce que** le matériau textile (2) présente un grammage entre 15 et 45 grammes par mètre carré (g/m²).

7. Élément composite selon l'une des revendications 1 à 6, **caractérisé en ce que** le support (1) est un film.

8. Élément composite selon l'une des revendications 7, **caractérisé en ce que** le film présente un motif de décoration imprimé.

9. Élément composite selon la revendication 7 ou 8,
**caractérisé en ce que** le film a une épaisseur entre 8 µm et 30 µm.

10. Élément composite selon l'une des revendications 1 à 9, **caractérisé en ce que** le support (1) et le matériau textile (2) ne sont pas reliés sur toute la surface par de l'adhésif, sachant que l'adhésif est appliqué en un motif de surfaces d'adhésif et de surfaces sans adhésif.

11. Procédé de formation d'un élément composite de fermetures auto-agrippante,
dans lequel un film en tant que support (1) est alimenté,
dans lequel un matériau textile (2) est alimenté, qui présente une structure de base produite par des brins (F₁, F₂) et des brins bouclés (S) intégrés par activation dans la structure de base, qui forment des boucles prévues pour être reliées à des crochets de crampon, et qui sont formés par un fil multi-filaments texturé présentant une finesse entre 35 dtex et 55 dtex et composé de 11 à 25 filaments,
sachant que le support (1) et le matériau textile (2) sont reliés entre eux en une bande de matériau par un adhésif appliqué en un motif de surfaces d'adhésif et de surfaces sans adhésif
sachant la structure de base est produite par des brins (F₁) d'un premier fil de base et des brins (F₂) d'un second fil de base et
sachant que des éléments composites individuels sont séparés de la bande de matériau.

12. Procédé selon la revendication 11, dans lequel le fil multi-filaments texturé des brins bouclés (S) est en outre rendu bouffant par air comprimé, brossage ou autre traitement mécanique du matériau textile après formation de la bande de matériau.

13. Procédé selon la revendication 11 ou 12, dans lequel les éléments composites individuels séparés de la bande de matériau sont disposés au niveau de la taille d'une couche-culotte.
